# EUROPEAN PATENT SPECIFICATION

(11) **EP 3 340 908 B1**
(45) Date of publication and mention of the grant of the patent: **20.05.2020**
(21) Application number: 16751591.5
(22) Date of filing: 16.08.2016
(51) Int. Cl.: A61B 17/54, A61B 17/00, A61B 17/30, A61B 17/32

(54) **SKIN-ABRADING SURFACE THAT RELEASES AGENTS TO THE SKIN**
HAUTABREIBENDE OBERFLÄCHE ZUR FREISETZUNG VON WIRKSTOFFEN AN DIE HAUT
SURFACE ABRASIVE SUR LA PEAU QUI LIBÈRE DES AGENTS À LA PEAU

(30) Priority: 26.08.2015 EP 15182447
(43) Date of publication of application: 04.07.2018
(73) Proprietor: Koninklijke Philips N.V., 5656 AG Eindhoven (NL)
(72) Inventor: HORTON, Margaret Ruth, 5656 AE Eindhoven (NL); BEIJENS, Linda Goverdina Maria, 5656 AE Eindhoven (NL); VAN AMERONGEN, Hendrik Halling, 5656 AE Eindhoven (NL); JURNA, Martin, 5656 AE Eindhoven (NL); PALERO, Jonathan Alambra, 5656 AE Eindhoven (NL); VULDERS, Roland Cornelis Martinus, 5656 AE Eindhoven (NL)
(74) Representative: Kabuk, Yavuz
(86) International application number: PCT/EP2016/069358
(87) International publication number: WO 2017/032634

(56) References cited:
- EP-A1- 2 444 015
- WO-A1-2011/006009
- DE-U1-202014 102 546
- US-A1- 2003 165 550
- US-A1- 2007 156 124
- US-A1- 2013 345 721
- US-B1- 6 299 620
- US-B1- 6 641 591

## Description

### FIELD OF THE INVENTION

The invention relates to a microdermabrasion device and to a method for the controlled removal of at least part of the stratum corneum with such device.

### BACKGROUND OF THE INVENTION

Microdermabrasion methods are known in the art. US2006/0253125, for instance, describes a method and system for performing microdermabrasion on a patient's skin. The method comprises providing a handpiece having a supply lumen, a return lumen, and distal cap having a window. The supply lumen and return lumen are operatively connected to a control system. A flow of crystals is introduced through the supply lumen. The flow of crystals is directed from the supply lumen at the window. The patient's skin is abraded with the crystals. The crystals are drawn in a proximal direction through the return lumen. A mode selection switch on the control system can be switched. An abrasive area is added to the handpiece. The abrasive area is positioned against a patient's skin. A suction force is applied through the return lumen to the patient's skin. The abrasive surface is moved across the patient's skin surface.

US2007/0156124 describes an apparatus for treating skin having a console with a user input device and a hand piece assembly. The hand piece assembly is configured to treat a skin. A fluid line provides fluid communication between the console and the hand piece assembly. A manifold system is coupled to the console and controlled by the user input device. The manifold system is configured to hold releasable a plurality of fluid sources and deliver fluid from at least one of the plurality of fluid sources to the hand piece assembly.

EP2444015 describes a device having an abrasion unit for contacting human skin during treatment. A grip housing has a drive for transmitting rotational movement on the abrasion unit. The abrasion unit has a carrier for accommodating an abrasion pill that is produced from a mixture of aluminum oxide crystals, powdery cosmetic active substances and binding agents by pressing. The abrasion unit is connected with the carrier, and the housing has a tubular treating section connected with a suction device by an extraction hose.

US2013345721 describes an article for mechanical skin resurfacing techniques that is suitable for transferring mechanical energy from a handheld device to skin placed in contact with the article. The article may be formed of a fibrous structure having a first major surface having associated therewith an adhesive system and a second major surface, generally opposite the first major surface. The second major surface is arranged and configured to reversibly engage a fastener of a motion-generating unit. This document also describes a coupling device for coupling a motorized apparatus to a disposable skin-contactable element. The coupling device includes a water-resistant first attachment and a second attachment for releasable affixing said article to a surface of the motion generation unit.

WO2011006009 describes a microdermabrasion device for treating skin comprising a hand piece assembly having a distal end and a proximal end. The hand piece assembly includes at least one delivery conduit and at least one waste conduit. The microdermabrasion device additionally comprises a tip configured to be positioned along the distal end of the hand piece assembly, wherein the tip is adapted to contact skin surface. In several embodiments, the tip comprises a lip, a first opening in fluid communication with the fluid delivery conduit and a second opening in fluid communication with the waste conduit In one embodiment, the device includes one or more abrasive elements positioned along a distal end of the tip, wherein the abrasive elements are configured to selectively remove skin In some embodiments, the delivery conduit is configured to selectively deliver at least one time-release material to the skin surface being treated.

US2003165550 describes a composition including a base and a plurality of abrasive particles. An apparatus including a head, and an applicator coupled to the head, the applicator having dimensions suitable for contacting localized areas of human skin is also described. A method including applying a composition to an area of human skin, the composition comprising a base and a plurality of abrasive particles, and manipulating the composition over the area of human skin with a handle-operated instrument is also mentioned in this document.

DE202014102546U1, also published as US2016106468, describes a microdermabrasion device comprising a vacuum system and a device tip, wherein the vacuum system comprises a channel with a channel inlet at the device tip, wherein the channel inlet is surrounded by a channel rim which facilitates gliding of the device tip over a skin, and wherein the device tip comprises a microdermabrasion zone configured remote from the channel inlet with a recession configured between the microdermabrasion zone and the channel rim.

US6641591 describes an instrument and technique for the removal of epidermal layers in a controlled manner utilizing a hand-held instrument with a working end that comprises (i) a vacuum aspiration system, (ii) a source for delivery of a sterile fluids or pharmacological agents to the skin; and (iii) a skin interface surface in the working end that has specially shape structure for abrading surface layers of the patient's epidermis as the working end is moved over the patient's skin while at the same time causing rapid penetration of the fluids into the skin for therapeutic purposes. Movement of the working end across the skin causes abrasion of the surface layers in a path over the patient's skin. The method of the invention may be used in a periodic treatment for the removal of superficial skin layers that enhances the synthesis of dermal collagen aggregates by inducing the body's natural wound healing response. The method of the invention creates more normal dermal architectures in skin with limited depths of skin removal by the series of superficial treatments that may be comparable to the extent of neocollagenesis caused by a deep skin removal treatment (e.g., CO₂ laser skin removal).

US6299620 describes a system for atraumatic removal of skin surface layers in a treatment to induce neocollagenesis in the dermis to reduce wrinkles and alter the architecture of the dermal layers. An embodiment of the system comprises (i) a hand-held instrument with a resilient working skin interface that carries microscopic diamond fragments for abrading the skin surface in a controlled manner; (ii) a fluid source for supplying sterile fluids to the skin interface for cleaning skin debris from the skin interface; and (iii) a negative pressure source for pulling fluid to the skin interface and thereafter aspirating fluid and skin debris from a treatment site. The skin interface is formed of a resilient material such as silicone to allow the working end to flex and atraumatically engage the skin surface as it is translated across a treatment site. The system also carries a disposable cartridge filled with fluid in the hand-held instrument. The method of the invention includes: (i) actuating a negative pressurization source communicating with the skin interface via a plurality of apertures therein to draw the skin into the concave abrasive architecture of the working end; (ii) translating the abrasive architecture across the treatment site to cut or abrade a skin surface layer; and (iii) contemporaneous with the cutting step, flowing a fluid (e.g., sterile water) generally about and across the abrasive architecture between an arrangement of inflow and outflow apertures to remove skin debris and hydrate the skin.

US2013345721 describes an article useful for mechanical skin resurfacing techniques is suitable for transferring mechanical energy from a handheld device to skin placed in contact with the article. The article may be characterized by its durable abrasiveness, compressibility, displacement, and/or surface roughness. The article may be formed of a fibrous structure having a first major surface having associated therewith an adhesive system and a second major surface, generally opposite the first major surface. The second major surface is arranged and configured to reversibly engage a fastener of a motion-generating unit. The document also describes a coupling device for coupling a motorized apparatus to a disposable skin-contactable element. The coupling device includes a water-resistant first attachment and a second attachment for releasably affixing said article to a surface of the motion generation unit.

### SUMMARY OF THE INVENTION

The "micro dermo abrasion" or "mircodermabrasion" (MDA) technique is being used to help the upper skin layer (the so called stratum corneum) to renew in a faster way than it would normally do. Traditionally, crystal microdermabrasion system contains a pump, a connecting tube, a hand piece, and a vacuum source. While the pump creates a high-pressure stream of inert crystals, like aluminum oxide, to abrade the skin, the vacuum removes the crystals and exfoliated skin cells. Instead of abrasion with particles in a gas stream, also a roughened surface, such as a diamond surface, of the tip of the device can be used. This is for instance known as (diamond) microdermabrasion. Unlike the crystal microdermabrasion system, the (diamond) microdermabrasion does not produce particles from crystals that may be inhaled into patients' nose or blow into the eyes. A subject's skin comprises a skin surface formed by a stratum corneum layer, an epidermis layer below the stratum corneum layer and a dermis layer below the epidermis layer.

The present invention especially relates amongst others to a microdermabrasion device with a stationary abrasion zone (i.e. no moving abrasive part), such as a diamond microdermabrasion device. However, in other exemplary embodiments, the present invention also relates to a microdermabrasion device wherein the microdermabrasion zone (or microdermabrasion area) maybe moving (i.e. not stationary).

Especially, the term "microdermabrasion" refers to the mechanical removal of the outer layer(s) of the skin and is a well-established skin treatment to improve the appearance and feel of the skin. The mechanisms for skin improvement can include thickening of the epidermis, stimulation of collagen production in the dermis, compaction of the stratum corneum, removal of dead surface skin cells and more radiant skin. As indicated above, microdermabrasion is often achieved by particulate matter accelerated into the skin, or bringing a rough or abrasive surface into contact with the skin, often with the aid of a vacuum. When particulate matter is accelerated into the skin, such microdermabrasion systems require reservoirs of solid particles in excess and are inconvenient for home use.

Thus, this invention is especially concerned with embodiments based on abrasive surfaces. The abrasive surfaces for microdermabrasion in the prior art often contain glued or fixed particles from a rigid, especially inert, material, such as diamond, or can comprise a bristle or brush. The harshness of the treatment or the degree of stratum corneum removal is usually controlled by adjusting the abrasive surface properties and the strength of the skin contact with the abrasive surface, for example by adjusting the vacuum.

The cosmetic outcome of microdermabrasion can be improved with the application of a cosmetic formula containing an active ingredient(s) during or after treatment. One possible mechanism is that the removal of the outer skin layer can facilitate improved percutaneous penetration of cosmetic active ingredients. Furthermore, the stimulation of skin cell renewal processes due to the microdermabrasion can (further) be complemented by e.g. the addition of active ingredients that support skin renewal processes.

For instance, it may be possible to use a microdermabrasion system that delivers active ingredients to the skin. However, a primary disadvantage of such microdermabrasion system that delivers active ingredients to the skin is the inconvenience of the dispensing systems and the requirement of a fluid formula. Large volumes of low-viscosity fluids containing active ingredients may have to be coupled to the microdermabrasion device, and may have to be actively pumped into the device. The reasons for the large volumes is that the microdermabrasion usually employs a vacuum to facilitate skin contact and remove debris, and the fluids placed into skin contact are typically rapidly pumped away and thus need to be constantly replenished in excess. If the vacuum contact is lost, fluid losses can occur and the delivery of the fluid can interfere with the vacuum. The large volumes usually require refilling often through an external reservoir and such systems are not easy to embody in a convenient handheld embodiment for home use. Hence, would active ingredients (herein also called "functional materials") be used in such systems, they would be used in excess and are therefore wasteful.

Hence, it is an aspect of the invention to provide an alternative microdermabrasion device, which preferably further at least partly obviates one or more of above-described drawbacks, and wherein especially an active material is applied in such a way that active material use is convenient (for the user) and active material waste is low.

The invention is disclosed in claims 1, 14 and 15 with preferred embodiments disclosed in the dependent claims. It is herein suggested to use a microdermabrasion treatment tip that has the dual-function of delivering active ingredients and of microabrading the skin. The invention especially includes an abrasive microdermabrasion treatment surface that contains active ingredients in a solid or crystalline form that are e.g. consumed, released or dissolved during use. Hence, in a first aspect the invention provides a microdermabrasion device ("device") comprising a microdermabrasion zone for abrading a part of a skin of a subject, wherein the microdermabrasion zone comprises a support material and abrasive structures at least partially associated with the support material, wherein the microdermabrasion zone comprises a releasable material, releasable comprised by the microdermabrasion zone, wherein the releasable material is a solid in air at 20 °C and at 1 bar, and wherein the releasable material comprises a functional material. As further also elucidated below, in the embodiments of the invention, said microdermabrasion zone (at least partially) perimetrically surrounds a channel inlet at an inlet zone of the device tip of the microdermabrasion device, or said microdermabrasion zone is (at least partially) perimetrically surrounded by said channel inlet. In embodiments of the invention, the microdermabrasion device comprises a stationary microdermabrasion zone (see also below). In the embodiments of the invention the microdermabrasion device comprises a vacuum system and a device tip, wherein the vacuum system is in fluid communication with a channel inlet at an inlet zone of the device tip, wherein the vacuum system is configured to apply a vacuum to the inlet zone, wherein the device tip further comprises said microdermabrasion zone (see further also below). In embodiments of the invention the microdermabrasion device comprises a heating system configured to heat the microdermabrasion zone (see also below). Hence, the presently proposed device may in specific embodiments not include a liquid delivery system including cavities or containers or conduits for storage and/or transport of liquid material (i.e. material that is liquid at 20 °C at 1 bar). The proposed device may especially be configured to release the releasable material as function of melting of the releasable material and/or of the support material due to heating of the microdermabrasion zone (see also below).

For instance for home use, it is ideal to have a system that can deliver active ingredients to the skin without employing large volumes of fluids or formulas or a fluid management system, which is possible with the presently proposed device. Because the active ingredients may in embodiments comprise the treatment surface itself, or are in embodiments embedded into the treatment surface, there is no need for fluid management to deliver the active ingredients to the skin. Because the ingredients are in a solid form, they may optionally also be capable of microdermabrading the skin. With the present device, it is possible to deliver active ingredients that are not optimally dissolved or dispersed in a fluid formula, such as antioxidants (vitamin C), colloidal metals, or agents that should have preserved structural integrity when delivered to the skin, such as polymers for filling and swelling the skin, or large macromolecules intended to adhere to the surface of the skin. Hence, the herein described releasable material may e.g. comprise one or more of a colloidal metal, an agent that has a preserved structural integrity when delivered to the skin, such as polymers for filling and swelling the skin, and large macromolecules intended to adhere to the surface of the skin.

As indicated above, there are a number of types of microdermabrasion devices. For instance, in exemplary embodiments not forming part of the present invention, the microdermabrasion device is configured to produce abrasive particles, propelled by a gas flow, for application to the skin (in or in front of the inlet zone) for abrading the skin. In further embodiments the microdermabrasion device comprises an area with abrasive material, which has an abrasive function when the microdermabrasion device is moved over the skin. Optionally, an exemplary microdermabrasion device may also include a moving, such as rotating and/or vibrating, element including abrasive material. In principle, for all these embodiments the invention may be useful, as in all cases a vacuum may be used to remove the material that is abraded, and optionally also for massage properties. Hence, in an exemplary embodiment not forming part of the present invention, the microdermabrasion device further comprises an abrading material system configured to provide in a gas flow abrading material to the microdermabrasion zone. The terms "abrasive material" and "abrading material" may substantially refer to the same material; particle properties of embodiments of such materials are defined below (see e.g. information concerning "particulate material"). Alternatively, (or optionally additionally), the microdermabrasion zone (of the microdermabrasion device) comprises a microdermabrasion zone comprising immobilized abrading material. Especially, the latter embodiment and variants thereon are described herein.

Here below, the device is described in more detail, often with reference to the device when being applied as microdermabrasion device to the skin of a subject. The term "subject" especially refers to a living human, having an average body temperature of about 37 °C. In general, the average skin temperature of a human is about 34 °C. However, the device and accompanying claims relate also to the device *per se.* Application or use of the device may include movement of the device over the skin (i.e. moving while physically being into contact with the skin, especially moving while a skin dome protrudes into the inlet zone), but application or use may also include a stationary use, i.e. the device is not moved over the skin (while staying physically into contact with the skin).

As indicated above, the abrasion zone has abrasive properties, such as due to microscopic structures that facilitate abrasion of the upper part of the skin. Such microscopic structures may for instance be selected from the group consisting of alumina structures, such as alumina particles, and diamond structures, such as diamond particles. These structures are e.g. comprised by the microdermabrasion zone, such as an abrasion rim, i.e. attached or part of the rim.

Especially, the microdermabrasion zone comprises abrasive structures, such as particulate material, attached ("associated") to the microdermabrasion zone, having mean dimensions in the range of 0.1-1000 µm, especially 1-1000 µm, such as 2-300 µm, like 5-80 µm or 120-200 µm. These dimensions may also apply when a gas flow with abrading particles is applied. Alternatively or additionally, the microscopic structures may for instance be selected from the group consisting of silicon carbide structures, such as silicon carbide particles, and metal nitride structures, such as metal nitride particles. Alternatively or additionally, the microscopic structures may for instance be selected from the group consisting of metal oxide structures, such as aluminum oxide particles and aluminum oxide structures (see also above). Further options of microscopic structures may for instance be selected from the group consisting of diamond structures (see also above), boron nitride structures, silicon carbide structures (see also above), glass beads, steel grit structures, other metal grit structures, zirconium oxide structures, and quartz structures. Combinations of different kind of structures, both in chemical composition and/or dimensions, may also be applied.

The abrasive structures are especially available in the microdermabrasion zone in a density in the range of 20-500 structures/mm². Especially, particles in the size of 2-200 µm are available in this density (either mobile or immobilized in a microdermabrasion zone). The term "structure" may in this context also refer to "particle".

The abrasive structures, such as particles, may e.g. be glued to a surface, to provide the microdermabrasion zone. However, alternative options are also possible. The abrasive structures may also be at least partially embedded in a material (support material). An abrasive surface can be made in many ways. Abrasive structures, such as particles, may be glued or metal-plated. An abrasive structure can also be made from a solid material by machining or sanding a material. A surface treatment by a laser is also possible. Also by inject molding an abrasive surface can be created. Hence, the abrasive structures, such as particles, are associated to the support material. The substrate material may e.g. comprise a metal, a polymer, a siloxanes material, an(other) inorganic material, such as pumice, etc.. The substrate material, herein also indicated as support material, may be porous (see also below).

In an embodiment according to the invention, the microdermabrasion zone is stationary, i.e. especially the microdermabrasion zone is not configured to move relative to the device. In yet another exemplary embodiment, the microdermabrasion zone may be able to move. For instance, the device may be configured to let the microdermabrasion zone vibrate. Optionally or additionally, the device may be configured to let the microdermabrasion zone rotate. Such rotation may also include a vibration movement, for instance when the rotation is only a small rotation hence and forth.

With such MDA device, at least part of the stratum corneum can be removed from the skin of a human. This can be done in a non-therapeutical treatment, such as a cosmetic treatment. Hence, the invention also provides in a further aspect a method for the controlled removal of at least part of the stratum corneum of a part of a skin of a subject and application of a functional material to said part of said skin of said subject, the method comprising contacting the microdermabrasion device as especially defined herein with the part of the skin, removing at least part of the stratum corneum while applying the functional material to the part of the skin, especially while applying a vacuum to the vacuum channel (see further also below).

The microdermabrasion zone is especially configured for abrading a part of the skin of the subject, during use of the device. Hence, when bringing the microdermabrasion zone, or at least part of it, in contact with the skin, and especially by moving the device over the skin, the part of the skin that is in contact with the microdermabrasion zone may be (at least partially) abraded. Though the disclosure does not exclude the use of abrading particles propelled by a gas flow for abrading the skin, the device at least comprises (immobilized) abrasive structures, such as having one or more dimensions of 0.1-1000 µm (see also above). The term "dimension" may especially refer to one or more of length, width, diameter, etc. The term may refer to the length, or width or diameter of an abrasive structure (such as particle), but it may also refer to a length, or width or diameter of a face or edge of an abrasive particle. For instance, facetted abrasive structures may be applied, such as crystalline material (see also below). Therefore, in embodiments the abrasive structures comprise one or more dimensions selected from the range of 0.1-1000 µm, such as 1-1000 µm, like 2-300 µm (see also above).

The microdermabrasion zone may be comprised by an element that is detachable from the microdermabrasion device. Such detachable element allows replacement of a releasable material depleted microdermabrasion zone. Hence, e.g. the tip of the device may be detachable, or part of the tip, i.e. the detachable element, may (thus) be detachable.

These abrasive structures may be attached to a support material or may be at least partially embedded in support material (see also above). For instance, the abrasive structures may be attached to a support material via one or more of mechanical attachment and chemical bonding, etc.. For instance, the structures may be glued to a support material. In yet another embodiment, the support material comprises an adhesive material. The term "associated" and similar terms may thus refer to a connection with the support material via the use of a glue, an adhesive, by at least partly embedding in the support material, etc.. The support material and the abrasive structures are especially the basis that may also facilitate the release of releasable material. This will be elucidated further below after having discussed some embodiments of the functional material and the releasable material.

As indicated above, the releasable material comprises the functional material. In embodiments, the releasable material essentially consists of the functional material. In other embodiments, the releasable material comprises the functional material and optionally one or more other materials. For instance, the releasable material may comprise a matrix for the functional material. For instance, the releasable material may comprise pumice or other type of material, such as another mineral, silicon carbide, alumina, etc. (or a combination of two or more of these), which comprises the functional material. The pumice, or other type of material, may function as host or matrix for the functional material.

In specific embodiments, the releasable material comprises one or more of (a) an abrasive material, (b) a cosmetic or skin care material, and (c) a pharmaceutical material. Hence, especially, the functional material comprises one or more of (a) an abrasive material, (b) a cosmetic or skin care material, and (c) a pharmaceutical material. Non-limiting examples of abrasive materials are given above.

In specific embodiments the functional material comprises a pharmaceutical material. Hence, the invention also provides a pharmaceutical material for use in a medical method wherein the device as described herein is applied to apply the pharmaceutical material to the subject (i.e. to part of the skin of the subject), with the pharmaceutical material especially comprised by the releasable material.

In yet further specific embodiment, the functional material comprises a cosmetic or skin care material. Therefore, the invention also provides a cosmetic method, wherein the device as described herein is applied to apply a cosmetic material to the subject, and wherein the cosmetic method comprises the herein described method wherein the functional material comprises, or essentially consists, of the cosmetic material (and especially essentially has only a cosmetic function). The phrase "cosmetic or skin care material" especially refers to substantially any substance capable of improving the condition and/or the appearance of the skin by affecting the skin's properties, especially the physical and/or chemical properties and/or biological properties.

Hence, the term "functional material" may also refer to a plurality of different functional materials.

In yet further specific embodiments, the functional material comprises one or more selected from the group consisting of a sugar amine, a vitamin (such as L-ascorbic acid), a phytosterol, an anti-wrinkle agent, an anti-atrophy agent, a flavonoid, an N-acyl amino acid compound, a retinoid, a peptide, an anti-cellulite agent, a desquamation agent, an anti-acne agent, an anti-oxidant, a radical scavenger, an anti-inflammatory agent, a tanning agent, a skin lightening agent, an antiperspirant agent, an anti-dandruff agent. Note that some of the materials described, or not described, herein may have more than one functionality.

Hence, especially the functional material is not specifically used for abrading the (part of the) skin, but may assist in abrading, or may assist in rejuvenation of the skin, or may have other useful purposes. Therefore, in specific embodiments the functional material comprises one or more of (a) a cosmetic or skin care material, and (b) a pharmaceutical material.

In further specific embodiments, the functional material comprises one or more selected from the group consisting of an anti-cellulite agent, a desquamation agent, an anti-acne agent, an anti-oxidant, a radical scavenger, an anti-inflammatory agent, a tanning agent, a skin lightening agent, a pigmentation modulation agent, an antiperspirant agent, an anti-dandruff agent, a moisturizing agent (may also be indicated as hydrating agent), a skin plumping (or swelling) agent, a skin structure restorative agent, a skin structure modulation agent, an anti-wrinkle agent, an anti-aging agent and a skin firming agent.

In yet further specific embodiments, the functional material comprises one or more selected from the group consisting of a sugar amine, a vitamin, such as L-ascorbic acid, a phytosterol, a flavonoid, an N-acyl amino acid compound, a retinoid, hyaluronic acid, and a peptide.

As indicated above, the support material and the abrasive structures are especially the basis that may also facilitate the release of releasable material. For instance, the abrasive structures may comprise the releasable materials. Alternatively or additionally, the releasable material is configured as abrasive structures. Further, the releasable material may be comprised by the support material. For instance, the releasable material may be embedded by the support material. Hence, in embodiments the support material may be configured as host or matrix for the releasable material. In yet further embodiments, the releasable materials is at least partially associated to the support material, but may not specifically be configured as abrasive material. Hence, the support material may comprise a surface comprising the abrasive structures and the releasable material. As also indicated below, embodiments may be combined.

Hence, in embodiments the releasable material is comprised by the abrasive structures. For instance, due to erosion (especially due to movement over the skin), melting (especially due to physical contact with the human skin and/or active heating), dissolving (due to physical contact with the human skin that may naturally have some moist or that may be made wet with water or another liquid (i.e. solvent for the releasable material)), etc., the releasable material is released, especially in a controlled way, such that over time the functional material may be subjected to the skin.

Alternatively or additionally, in embodiments the releasable material is comprised by the support material. Likewise, due to erosion (especially due to movement over the skin), melting (especially due to physical contact with the human skin and/or due to (active) heating of the microdermabrasion zone), dissolving (due to physical contact with the human skin that may naturally have some moist or that may be made wet with water or another liquid), etc., the releasable material is released, especially in a controlled way, such that over time the functional material may be subjected to the skin.

Also alternatively or additionally, in embodiments the microdermabrasion zone comprises releasable particulate material, wherein the releasable material is comprised by the releasable particulate material. The releasable particulate material is not necessarily designed as abrasive material, but may be distributed between the abrasive materials. Nevertheless, the releasable particulate material may be configured as abrasive material. In such embodiments, especially the particulate material comprises facetted material. Therefore, in further embodiments, the particulate material comprises crystalline material, even more especially consists of crystalline material. In such embodiments (also) the particulate material may comprise one or more dimensions selected from the range of 0.1-1000 µm. Hence, e.g. the facet(s) of the above indicated facetted material may have one or more dimensions selected from the range of 0.1-1000 µm, such as 1-1000 µm, such as 2-300 µm, like 5-80 µm or 120-200 µm.

As already indicated above, there may be different mechanisms that allow release of the releasable material. Especially, (a) erosion of part of the microdermabrasion zone due to application of the device on the skin, (b) melting of the releasable material and/or the support material (comprising the releasable material), due to contact with the human body and/or due to heating of the microdermabrasion zone, or (c) dissolving in or mixing with liquid on the skin, either available as natural liquid, such as especially sweat, or applied to the skin, are mechanisms that may free the releasable material that is otherwise solid in air at 20 °C and at 1 bar.

Especially, according to the invention, the releasable material is solid in air at 20 °C at 1 bar, wherein the air has a relative humidity selected from the range of 0-90%, such as 5-80% relative humidity. The relative humidity may e.g. be evaluated with a (sling) psychrometer, as known in the art. Especially, the relative humidity may be measured according to ASTM E337-02. Hence, in embodiments the releasable material is solid in air at 20 °C at 1 bar, having a relative humidity of 50%. Materials that are not solid, may leak away from the device, even when not being used.

According to the invention, the microdermabrasion device further comprises a heating system configured to heat the microdermabrasion zone. In this way, releasable material that may be released upon heating may be released (in a controlled way). Especially in such embodiment the releasable material may comprise a material that is solid in air at 20 °C at 1 bar but that may melt during operation, such as by heating of the releasable material to a temperature of selected from e.g. 40-65 °C, such as especially 40-50 °C.

Hence, in embodiments the device is configured to release the releasable material as function of one or more of (i) melting of the releasable material and/or of the support material due to contact of the microdermabrasion zone with a part of a skin of a subject and/or due to (active) heating of the microdermabrasion zone, (ii) dissolving or mixing of the releasable material and/or of the support material with a liquid on a part of a skin of a subject, (iii) eroding of the releasable material and/or of the support material due to contact of the microdermabrasion zone with a part of a skin of a subject. Additionally or alternatively, the device may be configured to release the releasable material as function of preferential bonding of the releasable material to the a part of a skin of a subject. For instance, due to covalent bonding or H-bonding with the skin, releasable material may bind to the skin (or a material thereon) and thereby be removed (and thus released) from the device. Hence, due to a kind of coating mechanism, i.e. coating of the skin, the releasable material is released from the microdermabrasion zone and sticks to the skin.

When the support material changes (such as by dissolving or melting or erosion, etc.), the releasable material (when comprised by the support) may be released.

After some use time, the microdermabrasion device may start getting depleted from the releasable material. Hence, in yet a further aspect the invention also provides a kit of parts comprising the microdermabrasion device as herein, wherein the microdermabrasion device comprises a detachable element comprising said microdermabrasion zone, wherein the kit further comprises a plurality of said detachable elements. This allows replacement of microdermabrasion zones which are depleted in releasable material. Hence, in yet a further aspect the invention also provides a detachable element *per se,* for use with the microdermabrasion device as described herein. The detachable element is configurable in a detachable way to the microdermabrasion device for functional use as microdermabrasion device as further described herein. Hence, the microdermabrasion device may include a receptor configured to receive the detachable element. The detachable element can functionally be coupled to the microdermabrasion device to provide the herein described microdermabrasion device with microdermabrasion zone with releasable material.

Especially, this invention provides an abrasive microdermabrasion treatment surface, where the abrasive element of the surface comprises active skin ingredients in a particulate or solid form that are capable of gradual consumption or release due to dissolution or mechanical wear. The ingredients are delivered to the skin as facilitate by microdermabrasion. The ingredients may be intended to penetrate the skin or remain on the surface of the skin for diverse skin benefits. This invention overcomes the disadvantage of a possible microdermabrasion system that delivers active ingredients, such as cosmetic ingredients that require fluid reservoirs and fluid delivery systems.

Especially, the microdermabrasion device comprises a vacuum system.

With such device, the massage function (especially caused by moving the device over the skin (and in contact with the skin) in combination with the vacuum) and abrasion function (especially caused by the microdermabrasion zone, optionally in combination with moving the microdermabrasion device over the skin (and in contact with the skin)) may optimally be executed.

The vacuum system may comprise a source of vacuum, such as pump, configured to provide a suction flow in a direction from the channel inlet to the source of vacuum, such as a pump. The channel inlet or inlet zone, which is at the device tip, is thus configured upstream of the vacuum pump. Especially, the device may be configured to provide a negative pressure ("underpressure") in the range of 5-80 kPa, such as especially 15-60 kPa, such as in the range of 20-40 kPa. This may especially imply that when the skin is in contact with the inlet zone, and closes off the inlet zone, the device is able to provide a pressure which is in the range of 15-60 kPa lower than atmospheric pressure. Hence, the term underpressure may especially indicate that when the skin is in contact with the inlet zone, the skin may be sucked at least partly into the inlet zone due to the suction of the vacuum system, leading to an underpressure in the inlet zone relative to ambient pressure. Hence, especially the vacuum system is configured to suck gas from the inlet zone away into the vacuum system.

In general, the inlet zone is configured in such a way, that a good closing connection with the skin may be achieved. Especially, the inlet zone comprises a rim, herein also indicated as "channel rim". This channel rim may be a (slightly) protruding part of the device tip. The channel rim may also be seen as a distal part or end part of the channel opening. Especially this rim will be in contact with the skin of a user during use of the device. Optionally, this rim may comprise the microdermabrasion zone with abrading material (see also below).

In embodiments the microdermabrasion device further comprises a user interface configured to allow a user select a user input parameter, such as especially related to a strength of the vacuum, and wherein the control unit is configured to control the vacuum (also) as function of the user input parameter. Alternatively or additionally, the control unit may configured to control the optional heating system and/or an optional actuator for vibrating and/or rotating the (part of the microdermabrasion device comprising the) microdermabrasion zone, etc..

Further, it may be desirable that the user may also influence the settings of the microdermabrasion device. For instance, the user may choose between e.g. options "light" and "intense", or "light", "medium", and "intense", or "high", "medium", and "low", etc., or the user interface may be configured to allow indicate the body zone that is to be treated, or the user interface may be configured to allow the user to select a specific program (e.g. face & arms), etc. etc.. The user interface may optionally comprise a graphical user interface. Further, optionally the user interface is a remote interface, such as an App on a smart phone, i-phone, tablet or other (portable) electronic device. With providing information via the user interface, the control unit may impose boundaries to the possibilities that may be chosen by the control unit when providing instructions to the vacuum system. For instance, the range of the vacuum setting may be limited and only a sub selection may be allowed (e.g. the stronger vacuums when choosing "intense" or "high").

Therefore, in the embodiments the microdermabrasion device comprises a vacuum system and a device tip, wherein the vacuum system is in fluid communication with a channel inlet at an inlet zone of the device tip, wherein the vacuum system is configured to apply a vacuum to the inlet zone, wherein the device tip further comprises said microdermabrasion zone.

As indicated above, especially in embodiments the microdermabrasion zone at least partially perimetrically surrounds said channel inlet or in embodiments said microdermabrasion zone is at least partially perimetrically surrounded by said channel inlet.

The term "perimetrically surrounding" and similar terms also include a surrounding of a perimeter having a (perimetrically) shape other than circular. Hence, e.g. the microdermabrasion zone may have a circular shape but may in embodiments also have an oval shape. Other shapes may also be possible. Further, the channel inlet may have a circular shape and the microdermabrasion zone may have an oval shape, surrounding the channel inlet. Yet further, the microdermabrasion zone may have a circular shape, surrounded by an oval channel inlet. Other shapes than circular and oval may also be possible. Further, as indicated above, the channel inlet and microdermabrasion zone do not necessarily have the same shape. The term "circular shape" and similar terms may indicate that the indicated item has a circular cross section.

The term "at least partially" (equivalent to "at least partly") and similar terms indicate that a first item that surrounds a second item may not necessarily surround the entire perimeter. By way of example, a square ("□") second item may be partially perimetrically surrounded with two straight ("|" and "|") or curved ("(" and ")") elements, forming the first item surrounding the second item (e.g. to provide a "|□|" configuration or a "(□)" configuration, etc.). Especially, the perimeter of the second item is at least perimetrically surrounded . The partial perimetrically surrounding may be a surrounding over in total at least about 180°, especially at least about 225°, even more especially at least about 270°, like in the range of 270-360°, like in the range of 315-360°, such as 360°. The fact that a first item at least partially perimetrically surrounds a second item may include physical contact between the first item and the second item, but they may also be configured remote from each other. Even part may be in contact and part may be remote.

More especially, in embodiments the channel inlet is surrounded by a channel rim, and the device tip comprises a microdermabrasion zone configured remote from the channel inlet with a recession configured between the microdermabrasion zone and the channel rim, wherein the channel inlet is (at least partially) perimetrically surrounded by the channel rim.

Especially, the microdermabrasion zone (at least partially) perimetrically surrounds the channel inlet, or optionally a (non-abrasive) channel rim. Especially, the channel inlet has a vacuum area in the range of 10-400 mm². Especially, the treatment head include a single channel inlet. Therefore, in embodiments the microdermabrasion device may comprise a single channel inlet (at least partially) perimetrically surrounded by the microdermabrasion zone, The device is especially configured to provide a negative pressure in the range of 5-80 kPa.

In alternative embodiments, the microdermabrasion zone is (at least partially) perimetrically surrounded by the channel inlet.

Especially, the channel inlet has a vacuum area in the range of 10-400 mm². Further, also in these embodiments the device may especially be configured to provide a negative pressure in the range of 5-80 kPa.

Further, the present device is especially a handheld device, which may include a rechargeable battery to use the device wireless. The device may include a plug or socket for a wired charging. The device may in embodiments not include a connection to an external vacuum, gas, or liquid delivery system. Hence, the device may include a vacuum system only, and not a system configured to provide a liquid (or a gas) to the skin of a user.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1d schematically depict some aspects of the MDA device;
Figs. 2a-2b schematically shows an embodiment where the active skin ingredient(s) itself comprises an abrasive skin surface and is capable of gradual consumption. The treatment tip can be connected to a larger device that is coupled to a vacuum pump to enable suctioning through an orifice;
Figs. 3a-3b schematically shows top and side views of treatment tip comprising a ring containing active ingredients in particulate form attached to a mounting piece. The treatment tip is connected to a device body that is coupled to a controlled vacuum through an orifice;
Fig. 4 schematically depicts a more detailed view of matrix of binding material containing active ingredient particulates with an air-solid interface comprising the abrasive surface;
Figs. 5a-5b schematically shows an embodiment for gradual release of active ingredient particulates due to gradual dissolution of the solid substance in which the particles are embedded and recession of air/solid interface;
Figs. 6a-6e schematically depicts some configurations of the releasable material; and
Figs. 7a-7b schematically depict some further configurations.
The schematic drawings are not necessarily on scale.

### DETAILED DESCRIPTION OF THE EMBODIMENTS

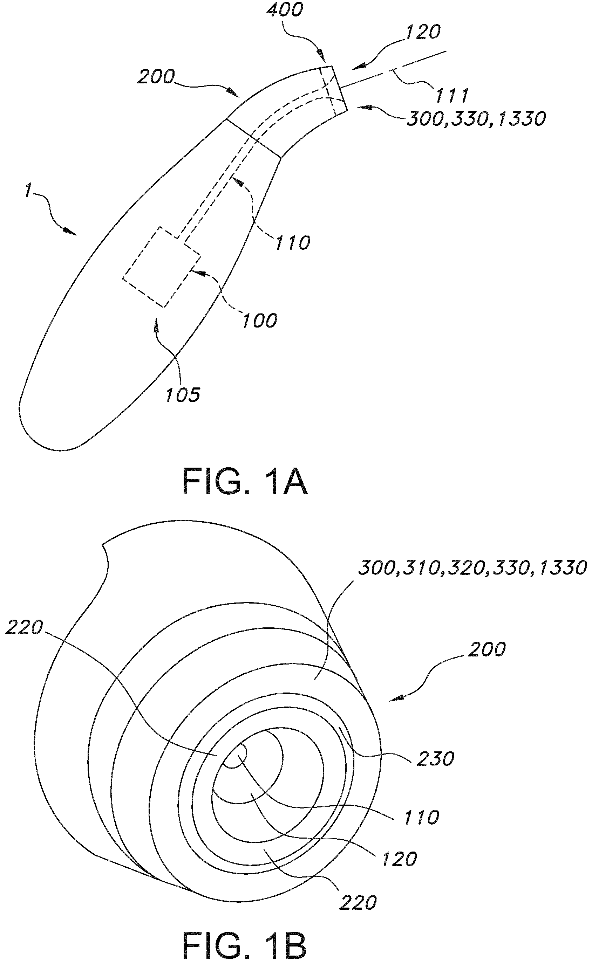

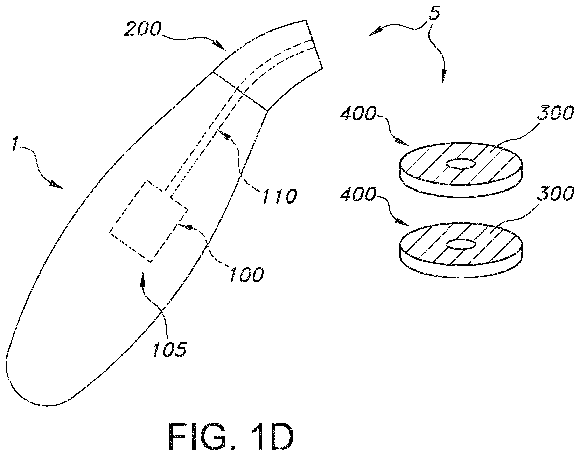

Figs. 1a and 1b schematically depict an embodiment of a microdermabrasion device 1. This device 1 comprises a vacuum system 100, with a pump 105 and a channel 110. Further, this device 1 comprises a device tip 200. Pump 105 can suck air into the channel 110. Channel 110 has a channel inlet 120 at the device tip 200. In other words, the device tip has a channel inlet 120 which is part of the channel 110 of the vacuum system 100. Here, the channel inlet 120 may be (perimetrically) surrounded by a channel rim 220 (though other embodiments may also be possible). This channel rim 220 may facilitate gliding of the device tip 200 over a skin (not shown). Hence, this channel rim 220 may be a non-abrasive rim. In embodiments, the device tip 200 further comprises a microdermabrasion zone 300 configured remote from the channel inlet 120. The microdermabrasion device 1 may comprise a detachable element 400 comprising the microdermabrasion zone 300; this is an optional embodiment, further also elucidated with the embodiment of Fig. 1d. The microdermabrasion zone comprises a functional material 1330. The functional material 1330, such as a cosmetic material, is comprised by a releasable material 330, which is especially solid under general non-use conditions. Optionally, the microdermabrasion device may further comprise a heating system (not depicted) configured to heat the microdermabrasion zone.

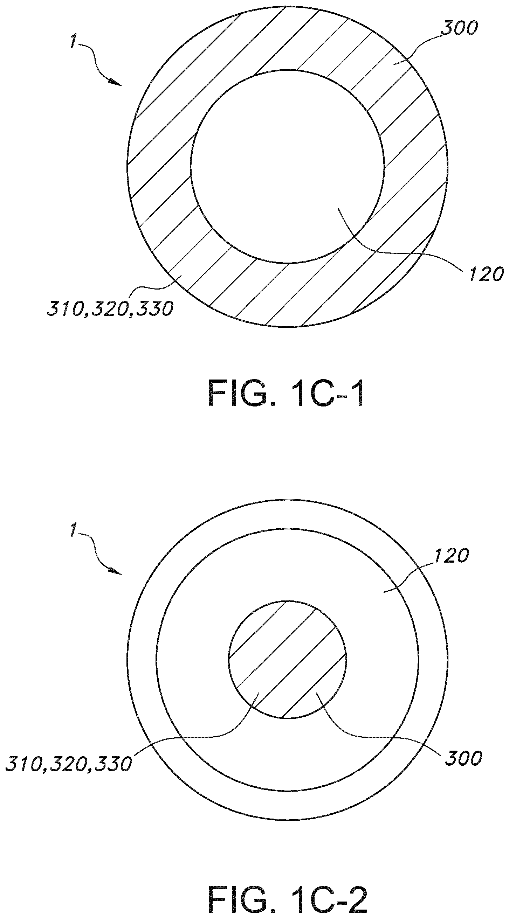

Optionally, the device tip 200 with the microdermabrasion zone 300 configured remote from the channel inlet 120 may further comprise a recession 230 configured between the microdermabrasion zone 300 and the channel rim 220. Fig. 1b schematically depicts in 3D view by way of example an embodiment of a device tip 200 having a ring-shaped channel rim 220, recession 230 and abrasion zone 300, all (perimetrically) surrounding the channel inlet 120, but the recession 230 remote from the channel rim 220 and the abrasion zone 300 remote from the channel rim 220 and recession 230. Reference 111 indicates a (virtual) channel axis. The microdermabrasion zone 300 may include abrasive structures (not depicted), which are known per se. The invention is not limited to the devices including such recession 230. In other embodiments, there may be a single rim comprising the microdermabrasion zone (see e.g. Fig. 1c-1).

Among the plurality of possible embodiments, especially using vacuum, there are amongst others two main embodiments. Fig. 1c schematically depicts top views of the tip of the device, showing the microdermabrasion zone 300. In the first embodiment, Fig. 1c-1, the channel inlet 120 is perimetrically surrounded by the microdermabrasion zone 300 (e.g. comprised by a rim); in the second embodiment, Fig. 1c-2, the microdermabrasion zone 300 is perimetrically surrounded by the channel inlet 120. Note that Fig. 1c-1 schematically depicts an embodiment without an intermediate non-abrasive rim. Substantially all embodiments described herein may also include the variant of Fig. 1c-2, though most of the embodiments are described and depicted herein in relation to Fig. 1c-1 and variants thereon (such as including an intermediate (non-abrasive) rim).

Fig. 1d schematically depicts a kit 5 of parts comprising the microdermabrasion device 1 as defined herein, wherein the microdermabrasion device comprises a detachable element 400 comprising said microdermabrasion zone 300, wherein the kit 5 further comprises a plurality of said detachable elements 400. Here, by way of example the microdermabrasion device 1 is schematically depicted with the detachable element 400 detached. Hence, the kit of parts is especially configured to assemble the herein described microdermabrasion device 1.

Amongst others, this invention provides a microdermabrasion treatment tip that has the dual-function of delivering active ingredients and microabrading the skin. The invention provides amongst others an abrasive microdermabrasion treatment surface that contains active ingredients in e.g. a solid or crystalline form that are consumed, released or dissolved during use. Because the ingredients are in a solid form, they are capable of microdermabrading the skin. Because the active ingredients comprise the treatment surface itself, or are embedded into the treatment surface, there is no need for fluid management to deliver the active ingredients to the skin.

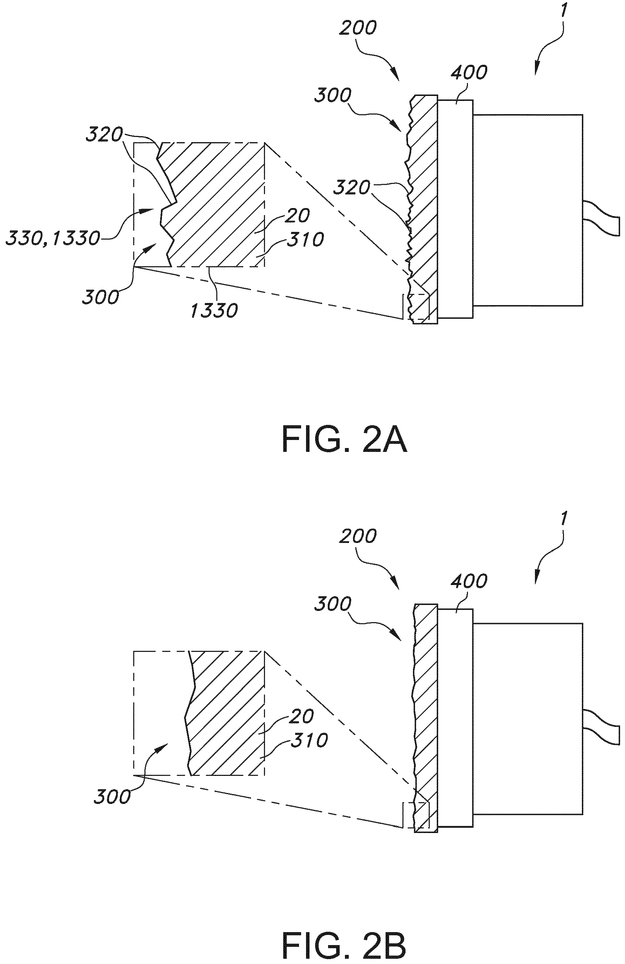

In an embodiment, the active ingredients comprise the treatment surface itself, i.e. the active ingredients are (also) configured as abrasive material. For this embodiment, solid-form active ingredients comprising at least a fraction of the abrasive ring outer surface are gradually consumed over time. The ingredient or ingredients are initially in a solid form that are capable of microdermabrasion and are capable of slow consumption. This embodiment is schematically illustrated in Figs. 2a-2b, wherein the skin abrasive surface can also become gradually smoother (at t₁ (>t₀) upon wear and consumption of the active ingredient, with Fig. 2a schematically depicting e.g. t₀ and with Fig. 2b schematically depicting t₁. Figs. 2a-2b schematically depict an embodiment where the active skin ingredient(s) itself comprises an abrasive skin surface and is capable of gradual consumption. The treatment tip can be connected to a larger device that is coupled to a vacuum pump to enable suctioning through an orifice. The device 1 may comprises a detachable element 400 comprising the microdermabrasion zone 300. In this way, after depletion, see right graph, the element may be replaced with a new well abrading microdermabrasion zone 300.

One consumption mechanism may be dissolution of the active ingredients due to contact with moisture present on the skin surface or below the skin surface. An example of such an active ingredient is vitamin C, which is a crystal that is known to dissolve in an aqueous environment. Another consumption method may be mechanical wear. If the abrasive surface is highly faceted with sharp protrusions, then protrusions with appropriate dimensions may be broken off when the surface comes in contact with the skin, while stable protruding parts may continue to abrade the skin while remaining intact. An example of such an active ingredient could be silicon (Si), that has the known property that it can be used to form microstructures through semiconductor processing techniques. The active ingredient can be consumed by a combination of these mechanisms.

The ingredients may themselves have a physical action on the skin, such as a scratching mechanical action, that enables them to penetrate into the skin barrier to facilitate dissolution due to exposure to interstitial fluid.

Preferably the ingredients naturally have a faceted outer surface texture to form a rough surface that is capable of removing skin tissue when the skin tissue is brought into contact with the surface via vacuum, such as salt or crystalline or semi-crystalline surface. The faceted outer surface can also be facilitated by molding processes, 3D printing or mechanical manipulation of the ingredients such as fracturing. As the ingredients are consumed, the faceted outer surface may gradually become smoother, so that the overall abrasive effect gradually becomes gentler.

The ingredients may have a surface cosmetic effect such as enhancing or changing the color or optical properties of the skin and be intended to stay on the surface of the skin, possibly binding to the skin surface. For this embodiment, the facets of the abrasion surface should may e.g. have a sizes in the scale of 0.1-1000 µm.

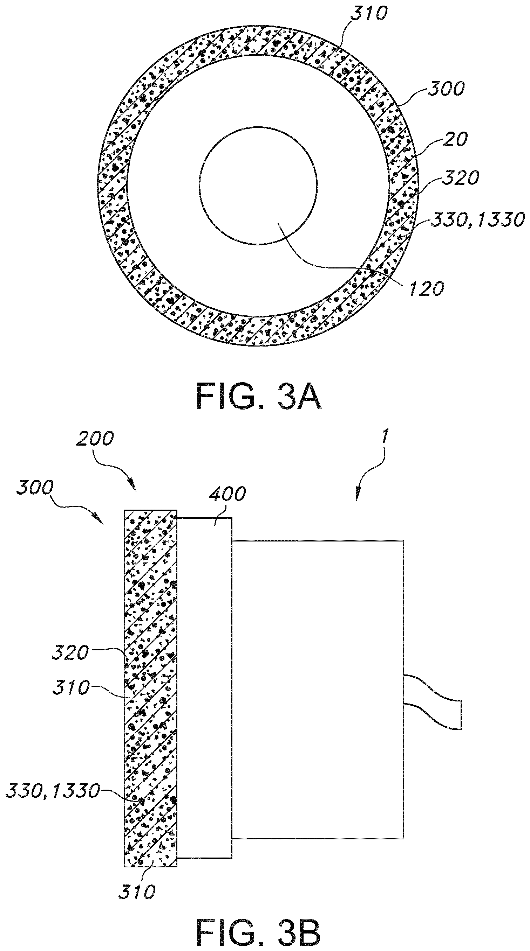

In yet further embodiments, the active ingredients may be embedded in the treatment surface. This embodiment and the envisioned device is schematically illustrated in Figs. 3a-3b (topview and sideview, respectively), the treatment surface can comprise a ring attached to a mounting piece whereby the ring and mounting piece comprise the treatment tip. The ring comprises in this case a matrix of rigid active cosmetic ingredients in particulate form. The treatment tip can be connected to a larger device that is coupled to a vacuum pump to enable suctioning through an orifice or channel inlet 120. In this way, a microdermabrasion zone 300 is provided wherein the support material 310 may comprise or host the releasable material 330, and also may at least partly enclose the abrasive structures 320. Hence, Figs. 3a-3b schematically depict top and side views of treatment tip comprising a ring containing active ingredients in particulate form attached to a mounting piece. The treatment tip is, by way of example, connected to a device body that is coupled to a controlled vacuum through an orifice.

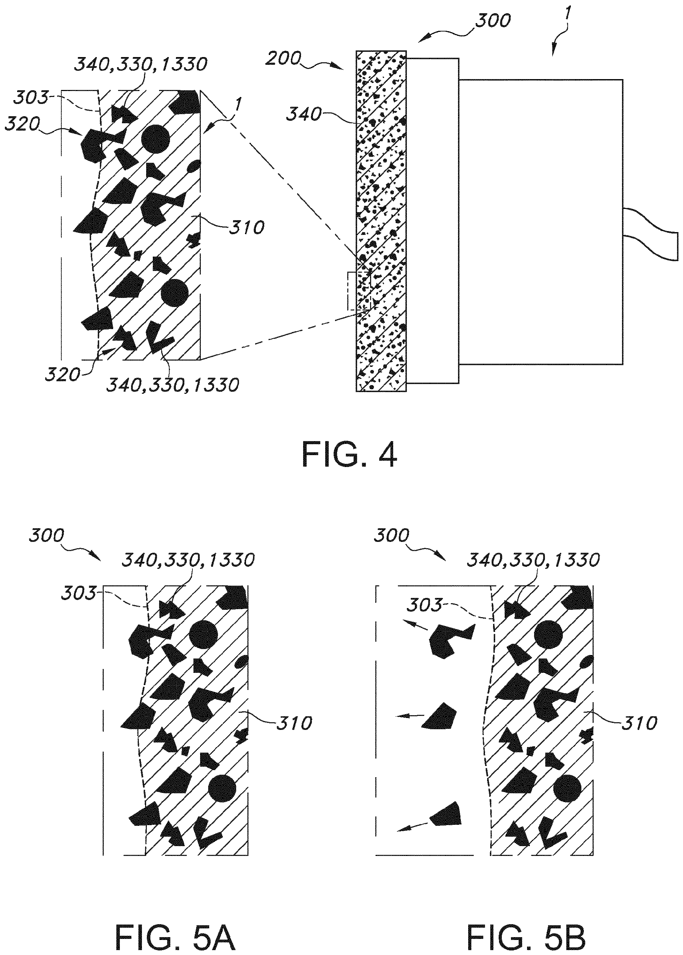

In a closer view of the outer surface of the skin abrading surface ring, schematically shown in Fig. 4, the active ingredient particulates (abrasive structures) are imbedded in a solid substance to form a matrix with an air/solid interface. Hence, Fig. 4 provides a schematically more detailed view of matrix of binding material containing active ingredient particulates with an air-solid interface 303 comprising the abrasive surface. In this embodiment the microdermabrasion zone 300 comprises releasable particulate material 340. Especially, in this embodiment the releasable material 330 is comprised by the releasable particulate material 340. For this embodiment, the solid substance can be configured to be gradually degraded over time, leading to release of the active ingredient particulates while simultaneously the air/solid interface recedes, as schematically illustrated in Figs. 5a-5b. These figures schematically show an embodiment for gradual release of active ingredient particulates due to gradual dissolution of the solid substance (support material) in which the particles are embedded and recession of air/solid interface.

The solid substance can be an adhesive material or composite material or cured material, etc. It may for example be a pumice with imbedded ingredients. The active ingredients can be capable of gradual dissolution, as described in the previous embodiment, or mechanical wear.

In addition to the active ingredient particulates, there may also be inert particulates such that are imbedded in the solid substance that are gradually released during use. Such particles can be used to intensify the function of microdermabrasion if they have defined sharp features. Thus, the active and inert particles are mixed together in the matrix.

This embodiment allows the possibility that the abrasive tip is self-renewing or that it is always sharp due to exposure of new particulates. Or the tip may be designed to have changing sharpness or roughness over time. This change can be facilitated by the density, the roughness, the shape, or the hardness of the particles, which can have varied properties according to their depth in the solid substance. Furthermore, the wear of the tip may be indicated for example by a color change, such that as the tip wears particles of a colored substance are revealed. For this embodiment, the active ingredient particles should have size 0.1 microns - 1 mm and are preferably in a solid form while imbedded in the solid material.

For all embodiments, possible ingredients include ascorbic acid (vitamin C), silicon, silica, and silicon derivatives, carbon-based materials, calcium and calcium derivatives, polymerized lactic acid such as poly-L-lactic acid. The ingredients can be a combination of ingredients derived from natural herbs and plants, may be sugars or salts or amino acids. The particulates may be a combination of bio-resorbable polymers and cosmetic active ingredients. The ingredients may be pigments or change the surface optical properties of the skin. In terms of skin benefits, the ingredients may enhance the effect of the microdermabrasion by having an anti-aging or skin rejuvenation effect. The ingredients may calm or sooth the skin or provide moisturisation. For all embodiments, the consumption of the active ingredients may be facilitated by the application of energy such as heat or light, which can be used to melt or change the physical state of the active ingredients. For example, poly-L-lactic acid can be heated to a glass transition temperature of around 60°C . Or ultrasonic energy may be used to disperse or facilitate dissolution of the active ingredients. For the matrix embodiment, the energy may be used to facilitate the degradation of the solid matrix. The dissolution can be triggered by abrasive ring contact with air, skin or a fluid such as water. For example, contact with water during shower may facilitate dissolution.

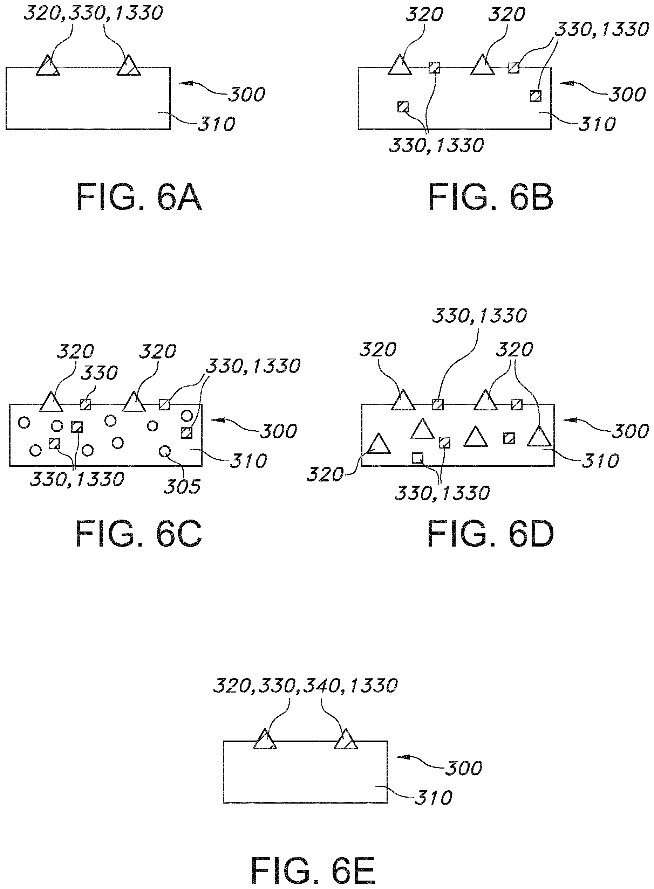

Figs. 6a-6e schematically depict some variants how the releasable material 330 can be implemented. Fig. 6a schematically shows that the abrasive structures 320 comprise the releasable material. For instance by erosion, the releasable material 330 can be released from the abrasive structures 320. Fig. 6b schematically depicts that the releasable material 330 is comprised by the support material 310. For instance by erosion, dissolution, melting, etc., the releasable material 330 may be released. Fig. 6c is a variant on the embodiment of Fig. 6b, but now the support material 310 comprises a porous material. For instance by dissolution, but also other mechanisms are possible, the releasable material 330 may be released from the support material 310. Fig. 3d is a further variant of embodiment of the embodiments of Fig. 6b or 6c. Here, the releasable material 330 is also distributed over the support material 310. Erosion of the support material 310 is necessary to release the material. As also abrasive structures 320 are distributed over the support material 310, erosion of the support material 310 may expose lower lying abrasive material 320 to the exterior, thereby maintaining abrasive properties over part of the release time. Fig. 6e schematically depicts an embodiment wherein the abrasive structures 320 are the releasable material 330; or, in other words, the releasable material 330 is configured as abrasive structures 320. Hence, the releasable material 330 may be configured or provided as particulate structures 340 which are configured as abrasive structures 320. Hence, the invention provides a skin-abrading surface that may release agents to the skin (during use of the microdermabrasion device comprising such skin-abrading surface, herein also indicated as microdermabrasion zone).

The present vacuum system can be used for crystal microdermabrasion system, which contains a pump, a connecting tube, a hand piece, and a vacuum source. While the pump creates a high-pressure stream of inert crystals, like aluminum oxide, to abrade the skin, the vacuum removes the crystals and exfoliated skin cells. Instead of abrasion with particles in a gas stream, the present invention is especially used for devices wherein a roughened surface, such as a diamond surface, of the tip of the device is be used. This is for instance known as (diamond) microdermabrasion. Here, a suction flow can be used for maintaining contact between the abrasion zone and the skin and/or for massage. The invention is especially described in relation to the later embodiment (see also the Figures).

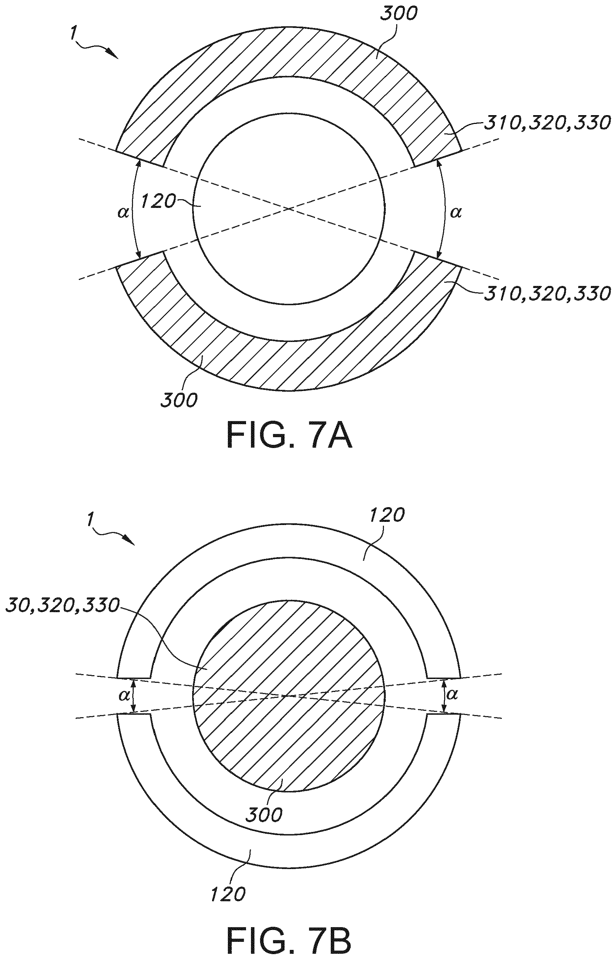

Figs. 7a and 7b are similar to those of Figs. 1c-1 and 1c-2. However, here the perimetrically surrounding is not entirely. Fig. 7a shows a microdermabrasion zone 300, configured remote from a channel inlet 120. Further, the microdermabrasion zone 300 partially surround the perimeter of this channel inlet 120. Angle α, which is by way of example identical on both sides, is about 35°. Hence, the microdermabrasion zone 300 perimetrically surrounds for 360°-2^{∗}35° = 290° the channel inlet 120. Here, by way of example the configuration is a "(O)" configuration. In fig. 7b the microdermabrasion zone 300 is perimetrically surrounded over less than 360° of the perimeter of the microdermabrasion zone 300. Here, the angle α, which is by way of example identical on both sides, is about 10°. Hence, the channel inlet perimetrically surrounds for 360°-2^{∗}10° = 340° the microdermabrasion zone 300. Note there may be one interruption or two interruptions, as schematically depicted, but also more than two interruptions, such as 4-12 interruptions. Anyhow, the partial perimetrically surrounding may especially be a surrounding over in total at least about 180°, especially at least about 225°, even more especially at least about 270°, like in the range of 270-360°, like in the range of 315-360°, such as 360°. In figs. 7a-7b the partial perimetrically surrounding is a surrounding over in total at least about 270°. Such conditions may (also) provide the desired effects described above.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims, which define the scope of the invention. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to a device comprising one or more of the characterizing features described in the description and/or shown in the attached drawings.

The various aspects discussed in this patent can be combined in order to provide additional advantages. Further, the person skilled in the art will understand that embodiments can be combined, and that also more than two embodiments can be combined.

## Claims

1. A microdermabrasion device (1) comprising a microdermabrasion zone (300) for abrading a part of a skin of a subject, wherein the microdermabrasion zone (300) comprises a support material (310) and abrasive structures (320) at least partially associated with the support material (310), wherein the microdermabrasion zone (300) comprises a releasable material (330), releasable comprised by the microdermabrasion zone (300), wherein the releasable material (330) is a solid in air at 20 °C and at 1 bar, wherein the releasable material (330) comprises a functional material (1330), the microdermabrasion device (1) comprising a vacuum system (100) and a device tip (200), wherein the vacuum system (100) is in fluid communication with a channel inlet (120) at an inlet zone (1200) of the device tip (200), wherein the vacuum system (100) is configured to apply a vacuum to the inlet zone (1200), wherein the device tip (200) further comprises said microdermabrasion zone (300), wherein said microdermabrasion zone (300) at least partially perimetrically surrounds said channel inlet (120) or wherein said microdermabrasion zone (300) is at least partially perimetrically surrounded by said channel inlet (120), wherein the microdermabrasion zone is stationary, and wherein the microdermabrasion device (1) comprises a heating system configured to heat the microdermabrasion zone (300).

2. The microdermabrasion device (1) according to claim 1, wherein the releasable material (330) comprises one or more of (a) an abrasive material, (b) a cosmetic or skin care material, and (c) a pharmaceutical material.

3. The microdermabrasion device (1) according to any one of the preceding claims, wherein the functional material (1330) comprises one or more selected from the group consisting of a sugar amine, a vitamin, a phytosterol, a flavonoid, an N-acyl amino acid compound, a retinoid, hyaluronic acid, a peptide, an anti-cellulite agent, a desquamation agent, an anti-acne agent, an anti-oxidant, a radical scavenger, an anti-inflammatory agent, a tanning agent, a skin lightening agent, a pigmentation modulation agent, an antiperspirant agent, an anti-dandruff agent, moisturizing agent, a skin plumping agent, a skin structure restorative agent, a skin structure modulation agent, an anti-wrinkle agent, an anti-aging agent and a skin firming agent.

4. The microdermabrasion device (1) according to any one of the preceding claims, wherein the device (1) is configured to release the releasable material (330) as function of melting of the releasable material (330) and/or of the support material (310) due to heating of the microdermabrasion zone (300).

5. The microdermabrasion device (1) according to any one of the preceding claims, wherein the releasable material (330) is comprised by one or more of the abrasive structures (320) and the support material (310).

6. The microdermabrasion device (1) according to any one of the preceding claims, wherein the abrasive structures (320) comprise one or more dimensions selected from the range of 0.1-1000 µm.

7. The microdermabrasion device (1) according to any one of the preceding claims, wherein microdermabrasion zone comprises releasable particulate material (340), wherein the releasable material (330) is comprised by the releasable particulate material (340), and, wherein the particulate material (340) comprises facetted material.

8. The microdermabrasion device (1) according to claim 7, wherein the particulate material (340) consists of crystalline material, and, wherein the particulate material (340) comprise one or more dimensions selected from the range of 0.1-1000 µm.

9. The microdermabrasion device (1) according to any one of the preceding claims, wherein the device (1) is configured to release the releasable material (330) as function of one or more of (i) melting of the releasable material (330) and/or of the support material (310) due to contact of the microdermabrasion zone (300) with a part of a skin of a subject, (ii) dissolving or mixing of the releasable material (330) and/or of the support material (310) with a liquid on a part of a skin of a subject, (iii) eroding of the releasable material (330) and/or of the support material (310) due to contact of the microdermabrasion zone (300) with a part of a skin of a subject.

10. The microdermabrasion device (1) according to any one of the preceding claims, wherein said microdermabrasion zone (300) perimetrically surrounds said channel inlet (120).

11. The microdermabrasion device (1) according to any one of the preceding claims, wherein the channel inlet (120) is surrounded by a channel rim (220), and wherein the device tip (200) comprises a microdermabrasion zone (300) configured remote from the channel inlet (120) with a recession (230) configured between the microdermabrasion zone (300) and the channel rim (220), wherein the channel inlet (120) is perimetrically surrounded by the channel rim (220), and wherein the microdermabrasion zone (300) perimetrically surrounds the channel rim (220), and wherein the device is configured to provide a negative pressure in the range of 5-80 kPa.

12. The microdermabrasion device (1) according to any one of the preceding claims 1-9, wherein the microdermabrasion zone (300) is perimetrically surrounded by the channel inlet (120).

13. The microdermabrasion device (1) according to any one of the preceding claims, wherein the channel inlet has a vacuum area in the range of 10-400 mm², wherein the microdermabrasion device (1) comprises a single channel inlet (120) perimetrically surrounded by the microdermabrasion zone (300), and wherein the microdermabrasion device (1) comprises a detachable element (400) comprising said microdermabrasion zone (300).

14. A non-therapeutic method for the controlled removal of at least part of the stratum corneum of a part of a skin of a subject and application of a functional material (1330) to said part of said skin of said subject, the method comprising contacting the microdermabrasion device (1) as defined in any one of claims 1-13 with the part of the skin, removing at least part of the stratum corneum while applying the functional material (1330) to the part of the skin.

15. A kit (5) of parts comprising the microdermabrasion device (1) as defined in any one of the preceding claims 1-13, wherein the microdermabrasion device (1) comprises a detachable element (400) comprising said microdermabrasion zone (300), wherein the kit (5) further comprises a plurality of said detachable elements (400).

## Patentansprüche

1. Ein Mikrodermabrasionsgerät (1), das einen Mikrodermabrasionsbereich (300) zum Abschaben eines Teils der Haut des Patienten umfasst, wobei der Mikrodermabrasionsbereich (300) aus einem Trägermaterial (310) und abrasiven Texturen (320) besteht, die zumindest teilweise mit dem Trägermaterial (310) verbunden sind, wobei der Mikrodermabrasionsbereich (300) aus einem lösbaren Material (330) besteht, das den Mikrodermabrasionsbereich (300) bildet. Hierbei handelt es sich beim lösbaren Material (330) um einen Feststoff in Luft bei 20 QC und 1 bar, wobei das lösbare Material (330) aus einem Funktionsmaterial (1330) besteht und das Mikrodermabrasionsgerät (1) ein Vakuumsystem (100) und eine Gerätespitze (200) umfasst. Hierbei besteht eine Fluidverbindung zwischen dem Vakuumsystem (100) und einem Kanaleinlass (120) im Einlassbereich (1200) der Gerätespitze (200), wobei das Vakuumsystem (100) so konfiguriert ist, dass es den Einlassbereich (1200) mit einem Vakuum beaufschlagt. Die Gerätespitze (200) besteht zudem aus dem angeführten Mikrodermabrasionsbereich (300), wobei der Mikrodermabrasionsbereich (300) den Kanaleinlass (120) zumindest teilweise kreisförmig umgibt, oder wobei der Mikrodermabrasionsbereich (300) zumindest teilweise kreisförmig vom Kanaleinlass (120) umgeben wird. Hierbei ist der Mikrodermabrasionsbereich unbeweglich, und das Mikrodermabrasionsgerät (1) umfasst ein Heizsystem, dass den Mikrodermabrasionsbereich beheizt (300).

2. Das Mikrodermabrasionsgerät (1) gemäß Anspruch 1, wobei das
lösbare Material (330) aus mindestens einem (a) abrasiven Material, einem (b) kosmetischen oder Hautpflegematerial und einem (c) pharmazeutischen Material besteht.

3. Das Mikrodermabrasionsgerät (1) gemäß einer der vorherigen Ansprüche, wobei das Funktionsmaterial (1330) aus mindestens einem Element der folgenden Gruppe besteht: Zuckeramin, Vitamin, Phytosterin, Flavonoid, N-Acyl-Aminosäure-Verbindung, Retinoid, Hyaluronsäure, Peptid, Anti-Cellulite-Mittel, Abschuppungsmittel, Anti-Akne-Mittel, AntiOxidationsmittel, Radikalfänger, entzündungshemmendes Mittel, Gerbmittel, Hautaufhellungsmittel, Mittel zur Modulation der Pigmentierung, schweißhemmendes Mittel, Antischuppenmittel, Feuchtigkeitsmittel, Mittel zur Hautauffüllung, Mittel zur Wiederherstellung der Hautstruktur, Mittel zur Modulation der Hautstruktur, Mittel gegen Falten, Mittel gegen Alterung und Mittel zur Hautstraffung.

4. Das Mikrodermabrasionsgerät (1) gemäß einer der vorherigen Ansprüche, wobei das Gerät (1) so konfiguriert ist, dass das lösbare Material (330) zum Schmelzen des lösbaren Materials (330) und/oder des Trägermaterials (310) durch das Erhitzen des Mikrodermabrasionsbereichs (300) freigegeben wird.

5. Das Mikrodermabrasionsgerät (1) gemäß einer der vorherigen
Ansprüche, wobei das lösbare Material (330) aus mindestens einer der abrasiven Texturen (320) und dem Trägermaterial (310) besteht.

6. Das Mikrodermabrasionsgerät (1) gemäß einer der vorherigen Ansprüche, wobei die abrasiven Texturen (320) aus mindestens einer Größenordnung im Bereich von 0,1-1.000 µm besteht.

7. Das Mikrodermabrasionsgerät (1) gemäß einer der vorherigen Ansprüche, wobei der Mikrodermabrasionsbereich aus einem lösbaren Aerosol (340) besteht. Hierbei besteht das lösbare Material (330) aus dem lösbaren Aerosol (340), während das Aerosol (340) aus Facettenmaterial besteht.

8. Das Mikrodermabrasionsgerät (1) gemäß Anspruch 7, wobei das Aerosol (340) aus kristallinem Material besteht. Hierbei besteht das Aerosol (340) aus mindestens einer Größenordnung im Bereich von 0,1-1.000 µm.

9. Das Mikrodermabrasionsgerät (1) gemäß einer der vorherigen Ansprüche, wobei das Gerät (1) so konfiguriert ist, dass das lösbare Material (330) freigegeben wird, um (i) das lösbare Material (330) und/oder das Trägermaterial (310) aufgrund des Kontakts des Mikrodermabrasionsbereichs (300) mit der Haut des Patienten zu schmelzen, um (ii) das lösbare Material (330) und/oder das Trägermaterial (310) mit einer Flüssigkeit auf der Haut des Patienten aufzulösen oder zu mischen, oder um (iii) das lösbare Material (330) und/oder das Trägermaterial (310) aufgrund des Kontakts des Mikrodermabrasionsbereichs (300) mit der Haut des Patienten zu erodieren.

10. Das Mikrodermabrasionsgerät (1) gemäß einer der vorherigen
Ansprüche, wobei der Mikrodermabrasionsbereich (300) den Kanaleinlass (120) kreisförmig umgibt.

11. Das Mikrodermabrasionsgerät (1) gemäß einer der vorherigen Ansprüche, wobei der Kanaleinlass (120) von einer Kanalfassung umgeben ist (220), und wobei die Gerätespitze (200) aus einem Mikrodermabrasionsbereich (300) besteht, der vom Kanaleinlass (120) durch eine Vertiefung (230) zwischen dem Mikrodermabrasionsbereich (300) und der Kanaleinfassung (220) getrennt ist. Hierbei ist der Kanaleinlass (120) kreisförmig von der Kanaleinfassung (220) umgeben, und der Mikrodermabrasionsbereich (300) umgibt die Kanaleinfassung (220) kreisförmig. Zudem ist das Gerät so konfiguriert, dass es einen Unterdruck im Bereich von 5 bis 80 kPa generiert.

12. Das Mikrodermabrasionsgerät (1) gemäß einer der vorherigen Ansprüche 1 bis 9, wobei der Mikrodermabrasionsbereich (300) kreisförmig vom Kanaleinlass (120) umgeben ist.

13. Das Mikrodermabrasionsgerät (1) gemäß einer der vorherigen Ansprüche, wobei der Kanaleinlass über einen Vakuumbereich im Bereich von 10-400 mm² verfügt. Hierbei besteht das Mikrodermabrasionsgerät (1) aus einem einzelnen Kanaleinlass (120), der kreisförmig vom Mikrodermabrasionsbereich (300) umgeben ist, wobei das Mikrodermabrasionsgerät (1) aus einem abnehmbaren Element (400) besteht, zu dem der Mikrodermabrasionsbereich (300) gehört.

14. Eine nicht therapeutische Methode für das kontrollierte Entfernen von mindestens einem Teil der Hornhaut von einem Teil der Haut eines Patienten sowie zum Auftragen eines Funktionsmaterials (1330) auf die Haut des Patienten, wobei die Methode daraus besteht, das Mikrodermabrasionsgerät (1) gemäß einer der Ansprüche 1 bis 13 mit der Haut des Patienten in Kontakt zu bringen, um mindestens einen Teil der Hornhaut zu entfernen, während das Funktionsmaterial (1330) auf die Haut aufgetragen wird.

15. Ein Teilesatz (5), der das Mikrodermabrasionsgerät (1) gemäß einer der Ansprüche 1 bis 13 umfasst, wobei das Mikrodermabrasionsgerät (1) aus einem abnehmbaren Element (400) besteht, das den Mikrodermabrasionsbereich (300) umfasst. Hierbei besteht der Satz (5) aus mehreren abnehmbaren Elementen (400).

## Revendications

1. Dispositif de microdermabrasion (1) comprenant une zone de microdermabrasion (300) pour abraser une partie d'une peau d'un sujet, dans lequel la zone de microdermabrasion (300) comprend une matière de support (310) et des structures abrasives (320) au moins partiellement associées à la matière de support (310), dans lequel la zone de microdermabrasion (300) comprend une matière libérable (330), libérable compris dans la zone de microdermabrasion (300), dans lequel la matière libérable (330) est un solide dans l'air à 20 °C et à 1 bar, dans lequel la matière libérable (330) comprend une matière fonctionnelle (1330), le dispositif de microdermabrasion (1) comprenant un système à vide (100) et une pointe de dispositif (200), dans lequel le système à vide (100) est en communication fluidique avec une entrée de canal (120) au niveau d'une zone d'entrée (1200) de la pointe de dispositif (200), dans lequel le système à vide (100) est conçu pour appliquer un vide à la zone d'entrée (1200), dans lequel la pointe de dispositif (200) comprend en outre ladite zone de microdermabrasion (300), dans lequel ladite zone de microdermabrasion (300) entoure au moins partiellement de manière périphérique ladite entrée de canal (120) ou dans lequel ladite zone de microdermabrasion (300) est entourée au moins partiellement de manière périphérique par ladite entrée de canal (120), dans lequel la zone de microdermabrasion est stationnaire, et dans lequel le dispositif de microdermabrasion (1) comprend un système de chauffage conçu pour chauffer la zone de microdermabrasion (300).

2. Dispositif de microdermabrasion (1) selon la revendication 1, dans
lequel la matière libérable (330) comprend au moins (a) une matière abrasive, et/ou (b) une matière cosmétique ou
de soin de la peau, et/ou (c) une matière pharmaceutique.

3. Dispositif de microdermabrasion (1) selon l'une quelconque des revendications précédentes, dans lequel la matière fonctionnelle (1330) comprend au moins un élément choisi dans le groupe constitué par une amine de sucre, une vitamine, un phytostérol, un flavonoïde, un composé d'acide aminé N-acylé, un rétinoïde, un acide hyaluronique, un peptide, un agent anticellulite, un agent desquamant, un agent anti-acnéique, un antioxydant, un fixateur de radicaux, un agent anti-inflammatoire, un agent de tannage, un agent éclaircissant la peau, un agent de modulation de la pigmentation, un agent antitranspirant, un agent antipelliculaire, un agent hydratant, un agent repulpant la peau, un agent restaurant la structure de la peau, un agent modulant la structure de la peau, un agent antirides, un agent antivieillissement et un agent raffermissant la peau.

4. Dispositif de microdermabrasion (1) selon l'une quelconque des revendications précédentes, dans lequel le dispositif (1) est conçu pour libérer la matière libérable (330) en fonction de la fusion de la matière libérable (330) et/ou de la matière de support (310) en raison du chauffage de la zone de microdermabrasion (300).

5. Dispositif de microdermabrasion (1) selon l'une quelconque des revendications précédentes, dans lequel la matière libérable (330) est constituée d'au moins une structure abrasive (320) et de la matière de support (310).

6. Dispositif de microdermabrasion (1) selon l'une quelconque des revendications précédentes, dans lequel les structures abrasives (320) comprennent au moins une dimension choisie dans la plage comprise entre 0,1 et 1 000 µm.

7. Dispositif de microdermabrasion (1) selon l'une quelconque des revendications précédentes, dans lequel la zone de microdermabrasion comprend une matière particulaire libérable (340), dans lequel la matière libérable (330) est constitué de la matière particulaire libérable (340), et, dans lequel la matière particulaire (340) comprend une matière à facettes.

8. Dispositif de microdermabrasion (1) selon la revendication 7, dans lequel la matière particulaire (340) est constituée d'une matière cristalline, et, dans lequel la matière particulaire (340) comprend au moins une dimension choisie dans la plage comprise entre 0,1 et 1 000 µm.

9. Dispositif de microdermabrasion (1) selon l'une quelconque des revendications précédentes, dans lequel le dispositif (1) est conçu pour libérer la matière libérable (330) en fonction (i) de la fusion de la matière libérable (330) et/ou de la matière de support (310) en raison du contact de la zone de microdermabrasion (300) avec une partie d'une peau d'un sujet, et/ou (ii) de la dissolution ou du mélange de la matière libérable (330) et/ou de la matière de support (310) avec un liquide sur une partie d'une peau d'un sujet, et/ou (iii) de l'érosion de la matière détachable (330) et/ou de la matière de support (310) en raison du contact de la zone de microdermabrasion (300) avec une partie d'une peau d'un sujet.

10. Dispositif de microdermabrasion (1) selon l'une quelconque des revendications précédentes, dans lequel ladite zone de microdermabrasion (300) entoure de manière périphérique ladite entrée de canal (120).

11. Dispositif de microdermabrasion (1) selon l'une quelconque des revendications précédentes, dans lequel l'entrée de canal (120) est entourée par un bord de canal (220), et dans lequel la pointe de dispositif (200) comprend une zone de microdermabrasion (300) conçue à distance de l'entrée de canal (120) dotée d'une récession (230) conçue entre la zone de microdermabrasion (300) et le bord de canal (220), dans lequel l'entrée de canal (120) est entourée de manière périphérique par le bord de canal (220), et dans lequel la zone de microdermabrasion (300) entoure de manière périphérique le bord de canal (220), et dans lequel le dispositif est conçu pour fournir une pression négative dans la plage comprise entre 5 et 80 kPa.

12. Dispositif de microdermabrasion (1) selon l'une quelconque des revendications 1 à 9 précédentes, dans lequel la zone de microdermabrasion (300) est entourée de manière périphérique par l'entrée de canal (120) .

13. Dispositif de microdermabrasion (1) selon l'une quelconque des revendications précédentes, dans lequel l'entrée de canal comporte une zone à vide dans la plage comprise entre 10 et 400 mm², dans lequel le dispositif de microdermabrasion (1) comprend une entrée de canal (120) unique entourée de manière périphérique par la zone de microdermabrasion (300), et dans lequel le dispositif de microdermabrasion (1) comprend un élément détachable (400) comprenant ladite zone de microdermabrasion (300).

14. Procédé non thérapeutique d'élimination contrôlée d'au moins une partie de la couche cornée d'une partie d'une peau d'un sujet et d'application d'une matière fonctionnelle (1330) à ladite partie de ladite peau dudit sujet, le procédé consistant à mettre en contact le dispositif de microdermabrasion (1), tel que défini dans l'une quelconque des revendications 1-13, avec la partie de la peau, à enlever au moins une partie de la couche cornée tout en appliquant la matière fonctionnelle (1330) à la partie de la peau.

15. Ensemble (5) de pièces comprenant le dispositif de microdermabrasion (1), tel que défini dans l'une quelconque des revendications 1-13 précédentes, dans lequel le dispositif de microdermabrasion (1) comprend un élément détachable (400) comprenant ladite zone de microdermabrasion (300), dans lequel l'ensemble (5) comprend en outre une pluralité desdits éléments détachables (400).
